Europäisches Patentamt

European Patent Office

Office européen des brevets

(11) Publication number: **0 273 485**
**A2**

## (12) EUROPEAN PATENT APPLICATION

(21) Application number: 87202305.6

(22) Date of filing: 24.11.87

(51) Int. Cl.⁴: **A23C 9/142** , A23L 1/304 , //A61K33/06,A23C9/13

(30) Priority: 25.11.86 NL 8602991

(43) Date of publication of application:
06.07.88 Bulletin 88/27

(84) Designated Contracting States:
BE DE FR GB NL

(71) Applicant: **Verenigde Coöperatieve Melkindustrie Coberco B.A.**
**Nieuwstad 69**
**NL-7201 NM Zutphen(NL)**

(72) Inventor: **Van den Boogaard, Cornelis**
**Hoefslag 4**
**NL-7213 BW Gorssel(NL)**

(74) Representative: **Smulders, Theodorus A.H.J. et al**
**Vereenigde Octrooibureaux Nieuwe Parklaan 107**
**NL-2587 BP 's-Gravenhage(NL)**

(54) **A process for preparing a raw material for human food; human food; a process for preparing a pharmaceutical composition.**

(57) A composition of matter useful in preventing or therapeutically treating health problems associated with calcium deficiency is prepared by subjecting milk, curd whey, casein whey or another dairy fluid in acid condition to ultrafiltration, isolating the resulting calcium-rich permeate, and optionally adding ingredients for improving or modifying taste, consistency, colour, flavour, turbidity and/or nutritional value. The calcium/protein ratio, expressed in mg Ca per g protein is preferably higher than 200, and in a preferred embodiment vitamin D is added to the resulting product in a proportion of 10-100 International Units per 100 g.

EP 0 273 485 A2

## A process for preparing a raw material for human food; human food; a process for preparing a pharmaceutical composition.

This invention relates to a process for preparing a raw material for human food and to a human food. It is well-known that milk contains a large number of important nutrients, and partly owing to its high nutrient density can contribute substantially to optimum human nutrition.

It is also well-known that calcium, in the high concentration in which it is present in milk, is of great dietetic importance.

It is increasingly being recognized that there is a correlation between the presence of calcium in the diet and some frequently occurring health problems. The most prominent of these is osteoporosis, or bone decalcification, although hypertension and cancer have also been mentioned as conditions which can be combatted by providing ample amounts of calcium.

Experts from various disciplines have pointed out that, especially elderly people threatened by osteoporosis should attempt to increase their uptake of calcium. Naturally, the use of milk is recommended for this purpose, but also calcium tablets are increasingly being used. The consumption of milk in the quantities desirable with regard to calcium uptake may be objection able because of problems of acceptance, undue protein consumption, excessive energy uptake, etc.

It is one object of the present invention to provide an article of food whereby the calcium present in milk can be offered for consumption in such a form as to satisfy the need of dietary calcium in the most effective manner.

It is an other object of the present invention to substantially increase the value of a by-product of the dairy industry.

Dairy processes essentially directed to produce protein, such as the preparation of cheese, casein and curds, produce whey as a residual product. This whey has been regarded as worthless and simply discarded for a long time. In the recent past, developments in production and environmental control have led to such upgrading processes, that, at present, virtually all whey is being used in Western Europe.

The most important uses of whey products are in cattle food, and are based largely on the protein present in whey. Deproteinized whey is almost only suitable for the preparation of lactose and represents a low value.

As a result of the recent development of membrane processes, processes directed to the production of protein now leave a protein-free residual product, namely, permeate, instead of whey.

This permeate, like the above deproteinized whey, only has a very low value. In the dairy industry, it is a recognized problem that the development of membrane processes is jeopardized because it is not known how the inevitable permeate can be upgraded.

The present invention provides a process for producing a raw material for human food, which is characterized by subjecting milk, curd whey, casein whey or another dairy fluid in acid condition to ultrafiltration, and isolating the resulting calcium-rich permeate.

According to the invention, the calcium/protein ratio in the resulting product, expressed in mg Ca per g protein is preferably higher than 200.

The invention also provides a process for preparing an agent for use in the prevention or therapy of osteoporosis and other health problems associated with calcium deficiency, which is characterized by subjecting milk, curd whey, casein whey, or another dairy fluid in acid condition to ultrafiltration, and isolating the resulting calcium-rich permeate.

The invention comprises the possibility of adding one or more agents for improving taste, consistency, colour, flavour, turbidity and/or nutrient value.

A preferred embodiment of the invention is characterized by adding vitamin D to the resulting product in a proportion of 10 - 100 International Units per 100 g.

Owing to the addition of vitamin D, which is of vital importance for a good calcium uptake, the anti-osteoporosis activity is enhanced. Vitamin D is a fat-soluble vitamin, which occurs in milk fat. As, however, the products produced by the process according to the invention are preferably low-energy products, which contain little, if any fat, the vitamin D will preferably be added separately.

The invention further provides a human food product consisting essentially of a calcium-rich permeate produced by the above process, and to which agents have been added to improve taste, consistency, colour, flavour, turbidity and/or nutrient value. The invention also provides an agent for use in the prevention or therapy of osteoporosis and other health problems associated with calcium deficiency, produced by the process described above.

The invention is based on the conception that, by using a membrane process, i.e., ultrafiltration, a permeate can be prepared which has an exceedingly high calcium content. In addition, this permeate has other properties which are dietetically desirable.

As this permeate further has the character of a semiproduct or raw material for composing articles of food, it can be offered in many forms, taking into account the specific requirements of certain groups of the population.

The invention is based on the known per se data that

-calcium in milk is bonded to milk protein and

-the bonding of calcium to protein is broken during acidification (cf Wong et al, J. Dairy Sci. 61, 1978, 1700-1703).

If now, the calcium-protein linkage is broken by fermentative or chemical acidification and subsequently the acid liquid is subjected to ultrafiltration, the calcium liberated will pass the ultrafiltration membrane and reach a relatively high concentration in the permeate.

The ultrafiltration of whey in the acidic condition, for that matter, has been described before, e.g., in the Japanese patent publication 60-232052 and in the French patent publication 2,425,809. The former publication, however, relates to the preparation of a minerals concentrate, which is produced by first stripping the acid whey from whey protein by ultrafiltration, and subsequently liberating the ultrafiltrate from lactose by further acidification with organic acid and concentration. The latter publication relates to the preparation of a product which can be used in the production of soft drinks, and is produced from the ultrafiltrate of acid whey by precipitation by adding a base.

The use of an ultrafiltrate of whey for preparing soft drinks is also known per se, for example, from Chem. Abstr. 101 (1984) No. 89156e and from German patent application 2,924,242. Both publications, however, only describe the ultrafiltration of non-acidic whey. It is true, that in the article referred to in Chem. Abstr. the starting product is acid cheese whey, but this is deacidified prior to ultrafiltration.

An important aspect of the invention resides in the calcium/protein ratio. It is known from nutritional physiology that the consumption of protein may result in a loss in calcium. Protein uptake is of course necessary, especially during growth. A surplus of protein, however, as is of frequent occurrence in the Western diet, leads to the excretion of nitrogen compounds via the kidneys, whereby calcium is lost too.

Therefore, a dietary advice directed against osteoporosis will not only seek to promote calcium uptake, but also to prevent excessive protein uptake.

In the following table, some analytical data are summarized which elucidate the present invention. The protein content is defined as $6.38 \times$ the content of nitrogen. The result is that protein appears to be present in the acid permeate, but actually the nitrogen here found will be present in a different form.

| | milk | acid permeate |
|---|---|---|
| dry solids (%) | 12.4 | 5.1 |
| protein (6.38 N; %) | 3.4 | 0.3 |
| lactose (%) | 4.6 | 3.9 |
| calcium (mg/100 g) | 120 | 120 |
| Ca/protein (mg/g) | 35 | 400 |
| Ca in dry solids (%) | 0.97 | 2.4 |

The table shows that the dry solids of the acid permeate contain 2.5 times as much calcium as do the dry solids of milk, while the calcium/protein ratio is increased still further.

The invention is illustrated in and by the following examples.

3

# 0 273 485

## Example 1

Acid whey was prepared by acidifying skim milk to pH 4.5 by fermentation with lactic acid bacteria and separating the curds. The resulting whey was subsequently pasteurized at 92°C for 15 seconds and subjected to ultrafiltration at 50°C in a DDS module 40, filter type GR 6. The resulting filtrate was cooled to 6°C. This filtrate contained 120 mg calcium per 100 g. The actual protein content was 0.04%, and there was an additional 0.26% of N containing non-protein components.

The ratio of calcium to protein (with all N containing components being regarded as protein) was therefore 407 mg/g.

## Comparative example 1

Fresh first cheese whey, obtained in the preparation of Gouda cheese, was fermented to pH 4.5, pasteurized and subjected to ultrafiltration in a manner similar to Example 1.

The filtrate only contained 46 mg calcium per 100 g. The protein content (calculated as total N $\times$ 6.38) was 0.36%; the calcium/protein ratio was therefore as low as 128.

## Example 2

Of the filtrate produced in accordance with Example 1, 75 parts were mixed with 25 parts yoghurt. The composition was homogenized at 20 kg/cm². The product was a pleasantly acid natural dairy drink containing 120 mg calcium and 1.1 g of protein per 100 g. The calcium/protein ratio was accordingly 110 mg/g.

## Example 3

Of the filtrate produced in accordance with Example 1, a refreshing clear drink was made with the following formulation

| | |
|---|---|
| acid whey filtrate (in accordance with Example 1) | 80% |
| concentrated apple juice (71° Brix) | 8% |
| saccharose | 5% |
| natural apple flavour | 0,7% |
| water | up to 100% |

This drink was filtered by means of a cellulose paper filter, heated at 95°C for some seconds, cooled to 20°C and aseptically bottled.

The calcium concentration of this drink was 98 mg/100 g, which with a protein concentration of 0.34% resulted in a calcium/protein ratio of 290 mg/g.

## Example 4

Of the filtrate produced in accordance with Example 1, 75 parts were mixed with 24 parts skim milk yoghurt and 1 part cream. This cream had been prepared by homogenizing 80 parts of pasteurized skim milk with 20 parts butter oil containing 0.015% of vitamin composition (Dohyfral $D_3$ 2000 AG). The composition was homogenized at 20 kg/cm².

There was thus obtained a dietary drink containing 120 mg calcium, 1.1 g protein and 60 International Units vitamin $D_3$ per 100 g, which is especially suitable as a health drink for elderly people.

## Claims

1. A process for producing a raw material for human food, characterized by subjecting milk, curd whey, casein whey or another dairy fluid in acid condition to ultrafiltration, and isolating the resulting calcium-rich permeate.

4

2. A process in claimed in claim 1, characterized in that the calcium/protein ratio in the resulting product, expressed in mg Ca per g protein is higher than 200.

3. A process as claimed in claim 1 or 2, characterized by adding vitamin D to the resulting product in a proportion of 10-100 International Units per 100 g.

4. A human food product consisting essentially of a calcium-rich permeate produced by a process as claimed in any of the preceding claims, to which agents have been added to improve taste, consistency, colour, flavour, turbidity and/or nutritional value.

5. A process for preparing an agent for use in the prevention or therapy of osteoporosis and other health problems associated with calcium deficiency, characterized by subjecting milk, curd whey, casein whey or another dairy fluid in acid condition to ultrafiltration, and isolating the resulting calcium-rich permeate.

6. A process as claimed in claim 5, characterized by adding vitamin D to the resulting product in a proportion of 10-100 International Units per 100 g.

7. A process as claimed in claim 5 or 6, characterized by adding one or more agents for improving taste, consistency, colour, flavour, turbidity and/or nutritional value.

8. An agent for use in the prevention or therapy of osteoporosis and other health problems associated with calcium deficiency, produced by the process as claimed in any of claims 5-7.